# EUROPEAN PATENT SPECIFICATION

(11) **EP 2 903 536 B1**
(45) Date of publication and mention of the grant of the patent: **05.07.2017**
(21) Application number: 13771202.2
(22) Date of filing: 27.09.2013
(51) Int. Cl.: A61B 17/02, A61B 17/15

(54) **LEG ALIGNMENT APPARATUS AND METHOD**
VORRICHTUNG UND VERFAHREN ZUR BEINAUSRICHTUNG
APPAREIL ET PROCÉDÉ D'ALIGNEMENT DE JAMBE

(30) Priority: 03.10.2012 GB 201217679
(43) Date of publication of application: 12.08.2015
(73) Proprietor: Corin Limited, Cirencester Gloucestershire GL7 1YJ (GB)
(72) Inventor: COLLINS, Simon, Tetbury, Gloucestershire GL8 8NT (GB); SABHARWAL, Harsimran, Bristol, Avon BS1 1DJ (GB); SUTCLIFFE, Andrew Craig, Nottingham, Nottinghamshire NG9 5HG (GB); GIROTTI, Gianluca, Cheltenham, Gloucestershire GL52 2JT (GB); BELLEMANS, Johan, B-3201 Langdorp (BE); VICTOR, Jan Maria Karel, B-8310 Brugge (BE); ELMSLIE, Ian James, Cirencester, Gloucestershire GL7 6HS (GB)
(74) Representative: Hocking, Adrian Niall
(86) International application number: PCT/GB2013/052528
(87) International publication number: WO 2014/053811

(56) References cited:
- EP-A2- 0 809 969
- WO-A1-99/35972
- WO-A1-2011/012533
- FR-A1- 2 949 315

## Description

The present invention relates to leg alignment apparatus for use in total arthroplasty of left and right knees. The invention may be used in a method of determining a distal end resection position for a femur of a knee joint. The closest prior art is document EP 0809969 A2, which defines the preamble of claim 1. Currently, it is common surgical practice to bring a varus or valgus knee back to neutral alignment during total knee arthroplasty. This, however, may not always be the most appropriate course of action, and it has been found that some patients and even athletes may have improved performance with a varus knee or alignment other than neutral.

Additionally, it is common practice to resect both the tibia and the femur to set the knee alignment. This can result in soft-tissue and/or alignment imbalance that may require further surgical resection or ligament release.

The present invention seeks to provide a solution to these problems.

According to the invention, there is provided leg alignment apparatus for use in total arthroplasty of left and right knees, the apparatus comprising first and second parts, the first part comprising an intramedullary rod, a femoral body for location on or adjacent to an anterior surface of a femur, a support element which extends or is extendable from the femoral body to the intramedullary rod for holding the femoral body stationary or substantially stationary relative to the intramedullary rod, a condylar locator which is pivotable relative to the femoral body and which is seatable on a distal end of the femur and a varus/valgus indicator, and the second part comprising a soft-tissue tensioning element to apply a separation between the femur and the tibia, characterised in that the first part includes a soft-tissue tensioning connector which is pivotably mounted on the femoral body, the soft-tissue tensioning element and the femoral body being pivotably and releasably interconnected or interconnectable via the soft-tissue tensioning connector, and in that the varus/valgus indicator indicates an angular alignment of the knee based on the intramedullary rod and the soft tissue tensioning connector. Preferable and/or optional features of the invention are set forth in claims 2 to 12, inclusive. The invention may be used in a method of determining a distal end resection position for a femur at a knee joint, the method comprising the steps of: a] prior to distal resection and with a leg in extension, providing a separation between a tibia and femur of the knee joint; b] with the leg still in extension and the separation of step a] applied, determining angular alignment of the knee based on an intramedullary rod extending along a femoral anatomical axis and/or a condylar plane and the separation between the tibia and femur; and c] determining a position of distal resection from a condylar plane of the femur.

The invention will now be more particularly described, by way of example only, with reference to the accompanying drawings, in which:
Figure 1 shows a perspective view of a first part of leg alignment apparatus, in accordance with the invention;
Figure 2 shows an exploded view of the part of the leg alignment apparatus, shown in Figure 1;
Figure 3 is a side perspective view from below of a soft-tissue tensioner forming a second part of the leg alignment apparatus, in accordance with the invention;
Figure 4 is a top plan view of the soft-tissue tensioner, shown in Figure 3;
Figure 5 shows a side view of a knee joint with the first part of the leg alignment apparatus located thereon, the second part being omitted for clarity;
Figure 6 shows an anterior plan of the knee joint with the first and second parts of the leg alignment apparatus located thereon;
Figures 7a to 7g show a neutral alignment technique, using the leg alignment apparatus, and Figures 7b and 7c show a soft-tissue tensioning element with a connector engager removed so that the meniscal compartment can be more easily viewed;
Figures 8a to 8g show a constitutional varus/valgus technique, using the leg alignment apparatus, and Figures 8b and 8c show a soft-tissue tensioning element with a connector engager removed so that the meniscal compartment can be more easily viewed; and
Figure 9a to 9g show a modified varus/valgus technique, using the leg alignment apparatus, and Figures 9b and 9c show a soft-tissue tensioning element with a connector engager removed so that the meniscal compartment can be more easily viewed.

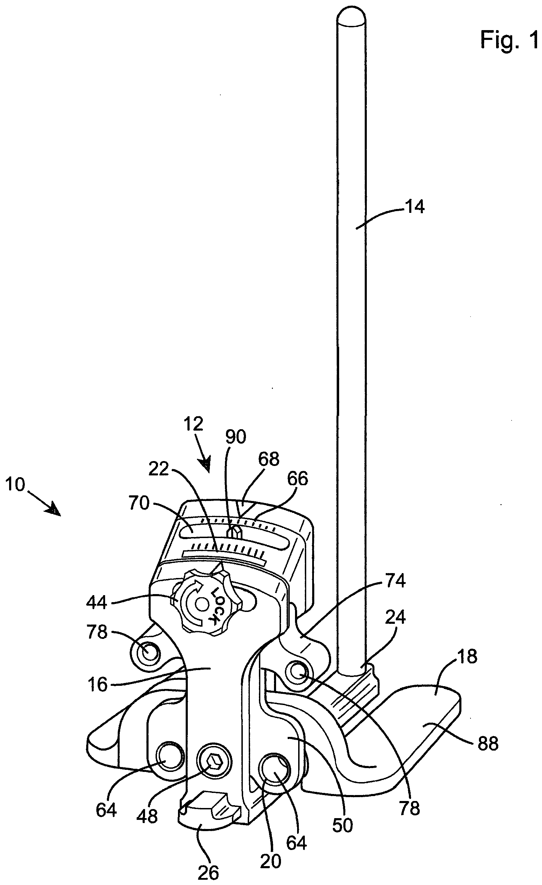

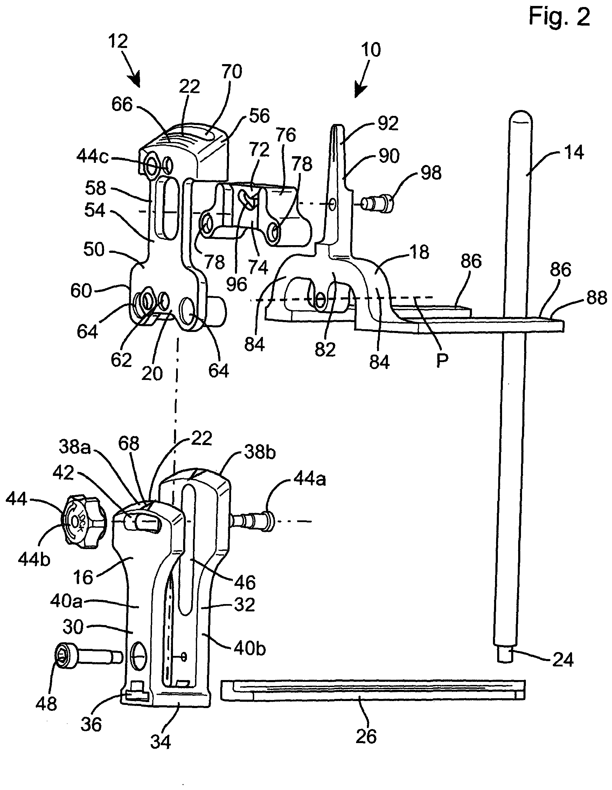

Referring firstly to Figures 1 and 2 of the drawings, there is shown a first part 10 of an embodiment of leg alignment apparatus 12 for use in total arthroplasty of both left and right knees 200. The first part 10 comprises an intramedullary rod 14, femoral body 16, condylar locator 18, soft-tissue tensioning connector 20, and a varus/valgus indicator 22.

The intramedullary rod 14 is an elongate shaft-like element which, following reaming, is insertable into the intramedullary canal 202 of a patient's femur 204. The intramedullary rod 14, once inserted, aids in defining the anatomical axis of the femur 204. Reaming and insertion in this case takes place from the distal end 206 of the femur 204 and with the knee 200 in flexion.

The intramedullary rod 14 is rigid and preferably linear, and may conveniently be an already known device.

A distal end 24 of the intramedullary rod 14 which extends from the distal end 206 of the femur 204 is connected to the aforementioned femoral body 16 by a support element 26. In this case, the support element 26 is a separate plate which is engagable with the intramedullary rod 14 to extend at or substantially at right angles to a longitudinal axis of the intramedullary rod 14. The support element 26 has a lateral extent which is suitable for extending in an anterior direction between the medial and lateral distal condyles 208 of the femur 204. The support element 26 is also preferably rigid and linear.

It should be understood that the support element 26 may be integrally formed as one-piece with the intramedullary rod 14, instead of being separable. It should also be understood that, although preferably at right angles with the longitudinal axis of the intramedullary rod 14, a longitudinal extent of the support element 26 may be at a non-perpendicular angle dependent on requirements. Furthermore, although preferably linear, a longitudinal extent of the support element 26 may be at least in part curved in at least one plane, again as necessity dictates.

The femoral body 16 in this embodiment is U-shaped in the plane of the longitudinal axes of the intramedullary rod 14 and the support element 26, providing a front plate 30, a rear plate 32 and a base plate 34 which interconnects the front and rear plates 30, 32. The base plate 34 includes a connector 36, which in this case is a keyway channel integrally formed as part of the base plate 34. The keyway channel 36 is adapted to receive as a sliding fit the support element 26, thereby providing adjustability along the support element 26 for accommodating different kinds of knee size. The femoral body 16 is holdable stationary or substantially stationary relative to the support element 26, and thus the intramedullary rod 14. In particular, the support element 26 prevents or limits any angular displacement of the femoral body 16 relative thereto.

The front and rear plates 30, 32 each includes a head portion 38a, 38b which tapers to a body portion 40a, 40b having a uniform or substantially uniform lateral extent along a majority of its longitudinal extent to the base plate 34. A longitudinal extent of the rear plate 32 is preferably greater than that of the front plate 30, which thereby allows the varus/valgus indicator 22 to be more easily read.

The head portion 38a of the front plate 30 includes a laterally extending slot 42 to slidably receive a locking element 44. The laterally extending slot 42 in this case is curved along its longitudinal extent. The locking element comprises a locking pin 44a having a locking head 44b at its proximal end. A distal end of the locking pin 44a is receivable in a locking aperture 44c of the soft-tissue tensioning connector 20. The laterally extending slot 42 preferably includes teeth or indentations along longitudinal sides thereof. Spacings between the teeth or indentations are complementarily shaped to receive diametrically-opposing locking fins or teeth formed on an underside of the locking head 44b. These spacings beneficially match a scale of the varus/valgus indicator 22. Other locking means can also be considered, for example, the locking pin being screw-threaded to frictionally engage the locking head with the front plate 30.

A longitudinally extending slot 46 is also provided centrally on the rear plate 32, and this extends from the head portion 38b to partway along the body portion 40b.

The soft-tissue tensioning connector 20 is pivotably received in the gap between the front plate 30 and the rear plate 32 of the femoral body 16. A pivot axle 48 extends between the front and rear plates 30, 32 and through the soft-tissue tensioning connector 20, thereby allowing relative angular displacement.

The soft-tissue tensioning connector 20 forms part of a soft-tissue tensioning element 50 of the apparatus 12, which also includes a soft-tissue tensioner 52 to be described hereinafter.

The soft-tissue tensioning connector 20 comprises a waisted plate-like body 54 having a head element 56, body element 58 and foot element 60. The soft-tissue tensioning connector 20 is preferably integrally formed as one-piece. The foot element 60 comprises a centrally located pivot-axle aperture 62, and a soft-tissue tensioner aperture 64 to each side of the pivot-axle aperture 62. The foot element 60 has a width which positions the soft-tissue tensioner apertures 64 outboard of the front plate 30 of the femoral body 16.

The head element 56 of the soft-tissue tensioning connector 20 together with the at least one of the head portions 38a, 38b of the front and rear plates 30, 32 of the femoral body 16 form the varus/valgus indicator 22. A scale 66 is provided, in this case, on the head element 56 of the soft-tissue tensioning connector 20 and a pointer 68 is provided on the upper end of the front plate 30 and the rear plate 32. However, the scale 66 could be provided on one or both of the head portions 38a, 38b of the front and rear plates 30, 32, and one or more pointers 68 could be provided on the head element 56 of the soft-tissue tensioning connector 20.

The head element 56 of the soft-tissue tensioning connector 20 is enlarged relative to its body element 58 in an axial direction of the pivot axle 48. This allows for a pointer slot 70 through the head element 56 in a plane which is parallel to the body element 58 of the soft-tissue tensioning connector 20.

The body element 58 of the soft-tissue tensioning connector 20 is waisted to be accommodated in a body channel 72 of a cutting-jig guide 74. The cutting-jig guide 74 is not required in order to determine an angular alignment of the knee 200 based on the intramedullary rod 14 and the soft-tissue tensioning element 50. However, the cutting-jig guide 74 is extremely beneficial for this invention, allowing a predetermined amount of bone to be resectable from a distal end 206 of the femur 204 based on the most prominent femoral distal condyle 208, as will be understood hereinafter.

A body member 76 of the cutting-jig guide 74 includes the body channel 72 and two outboard cutting-jig locators 78, which in this case are apertures but which may be any suitable locator, such as pins or markers. With the body element 58 of the soft-tissue tensioning connector 20 located in the body channel 72, the cutting-jig guide 74 is slidable longitudinally along and in parallel with the body element 58 of the soft-tissue tensioning connector 20.

Although preferably being a cutting-jig guide 74, so that a cutting jig 80 can be subsequently positioned on a distal end portion 210 of the femur 204, a cutting jig itself may be utilised in place of the cutting-jig guide 74. Such a cutting jig could feature openings, such as slots, for receiving a surgical saw blade to allow the bone resections to be performed without the cutting-jig guide 74.

A body 82 of the condylar locator 18 is also provided in the gap between the front and rear plates 30, 32 of the femoral body 16, pivotably mounted to the pivot axle 48 so as to be interposed between the foot element 60 of the soft-tissue tensioning connector 20 and the rear plate 32 of the femoral body 16. The condylar locator 18 is thus pivotable relative to the femoral body 16 and the soft-tissue tensioning connector 20.

An outrigger 84 extends laterally from each side of the body 82 of the condylar locator 18, and a condylar platform 86 extends in the pivot axis direction from a distal end of each outrigger 84. A condylar abutment surface 88 presented by each condylar platform 86 extends towards the intramedullary rod 14, and the condylar abutment surfaces 88 are coplanar. The condylar abutment surfaces 88 are adapted for seating on their respective medial and lateral distal condyles 208 of the femur 204.

A ligament tension indicator 90 is also provided as part of the condylar locator 18 and the soft-tissue tensioning element 50. The ligament tension indicator 90 includes a pointer arm 92 which extends upwardly from the body 82 of the condylar locator 18 to be movably received in the pointer slot 70 of the head element 56 of the soft-tissue tensioning connector 20. A ligament-tension scale 94 is provided on the head element 56, although it is feasible that the ligament-tension scale 94 could be provided on the pointer arm 92 and an indicator could be provided on the head element 56.

The pointer arm 92 is also connected to the body member 76 of the cutting-jig guide 74. The body member 76 includes a central V-shaped slot 96, and a runner 98 in the form of a pin extends in the direction of the pivot axis P from the pointer arm 92 to be slidably received in the V-shaped slot 96. This arrangement allows the cutting-jig guide 74 or cutting jig if integrally provided to be slidable longitudinally in unison with pivoting movement of the condylar locator 18. In this way, from the pointer arm 92 being at top-dead centre or zero degrees, a distance between a condylar platform 86 and its adjacent outboard cutting-jig locator 78 remains constant as the condylar locator 18 is pivoted and the cutting-jig locator 78 linearly slides.

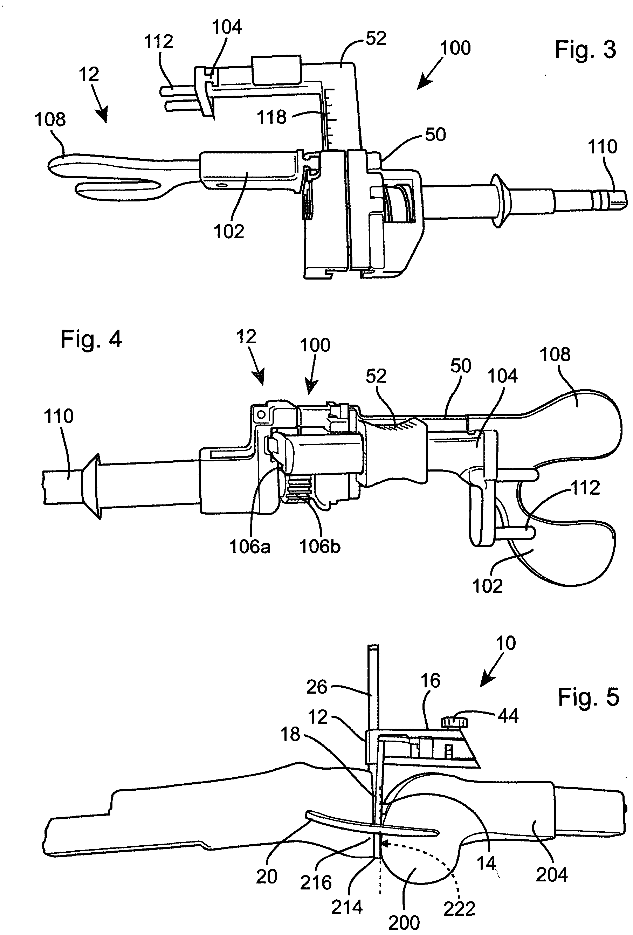

Referring to Figures 3 and 4, a second part 100 of the leg alignment apparatus 12 is shown. This second part 100 is the soft-tissue tensioner 52, engaging with the tibia and the soft-tissue tensioning connector 20 to provide tibio-femoral separation thereby tensioning both medial and lateral collateral ligaments 212.

The soft-tissue tensioner 52 comprises a tibial-platform element 102 and a connector engager 104 which is connected to the tibial-platform element 102 by a rack and pinion 106a, 106b. A tibial platform 108 is provided as part of the tibial-platform element 102, and extends at least substantially in parallel with the connector engager 104. The pinion 106b is drivable by a shaft 110 which is rotatable about its longitudinal extent, thereby driving the rack 106a and thus raising and lowering the connector engager 104.

The connector engager 104 in this embodiment comprises two spaced apart pins 112 which are receivable in the soft-tissue tensioner apertures 64. The pins 112 preferably extend in parallel or substantially parallel with the tibial platform 108.

Although a rack and pinion is utilised, a scissor-jack spreader mechanism, or any other suitable spreader mechanism may be utilised. For example, one or more shims may be used to distract the tibia and femur alone or in combination with the rack and pinion, scissor-jack spreader mechanism, or other spreader mechanism.

The apparatus 12 is preferably formed of biocompatible metal, such as titanium or stainless steel.

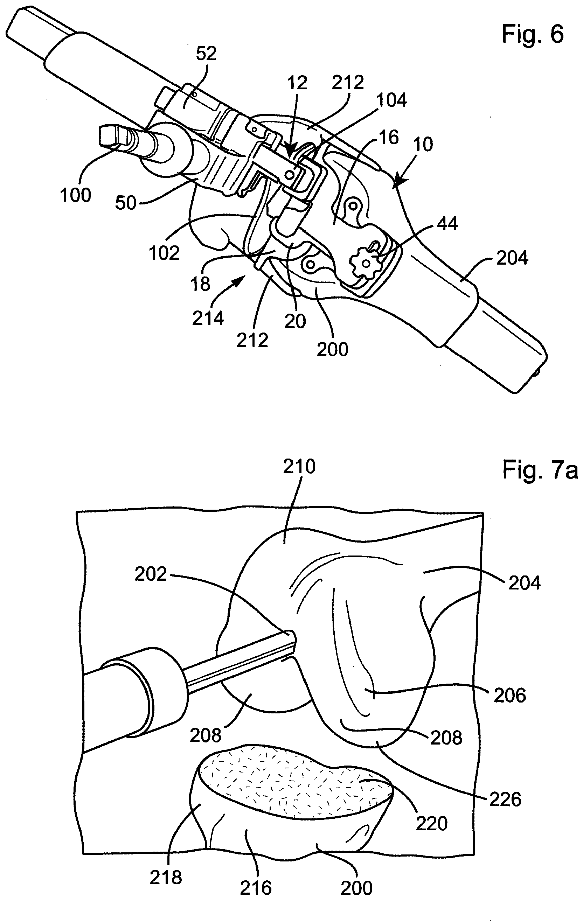

Referring to Figures 5 and 6, the apparatus 12 is shown applied to the left or right knee joint 214. With the knee 200 in flexion and a bore formed in the intramedullary canal 202, the intramedullary rod 14 is inserted and the support element 26 is attached. The knee joint 214 is then placed in extension, and the femoral body 16 is attached to the support element 26. The rear plate 32 of the femoral body 16 thus lies on or adjacent to the anterior surface of the distal end 206 of the femur 204, and the condylar platform 86 seats on at least the most prominent distal femoral condyle 208.

The tibial platform 108 of the soft-tissue tensioner 52 engages the proximal end of the tibia 216, and the connector engager 104 engages the soft-tissue tensioning connector 20.

To provide a, preferably linear, separation between the tibia 216 and the femur 204, the soft-tissue tensioner 52 is operated to move the connector engager 104 away from the tibial platform 108. This movement causes the condylar abutment surface 88 to be urged against the or each seated condyle 208.

Referring to Figures 7a to 7g, 8a to 8g, and 9a to 9g, three methods of determining a distal end resection position for a femur 204 at a knee joint 214 will now be described.

Figures 7a to 7g show a neutral leg alignment technique. It is preferable that a pre-operative examination of the knee joint 214 is first conducted, typically by X-ray. This allows preoperative determination of an existing angle between the anatomical axis and the mechanical axis of the femur 204, and whether the leg is in varus, valgus or neutral alignment.

As shown in Figure 7a and with either the left or right knee 200 in flexion, a proximal end 218 of the tibia 216 is resected perpendicularly or substantially perpendicularly to the mechanical axis of the tibia 216 in a coronal plane and preferably with a three degree posterior slope in the sagittal plane. Although three degrees is suggested, this may be in the range of zero to ten degrees.

The femoral intramedullary canal 202 is also reamed to accept the intramedullary rod 14.

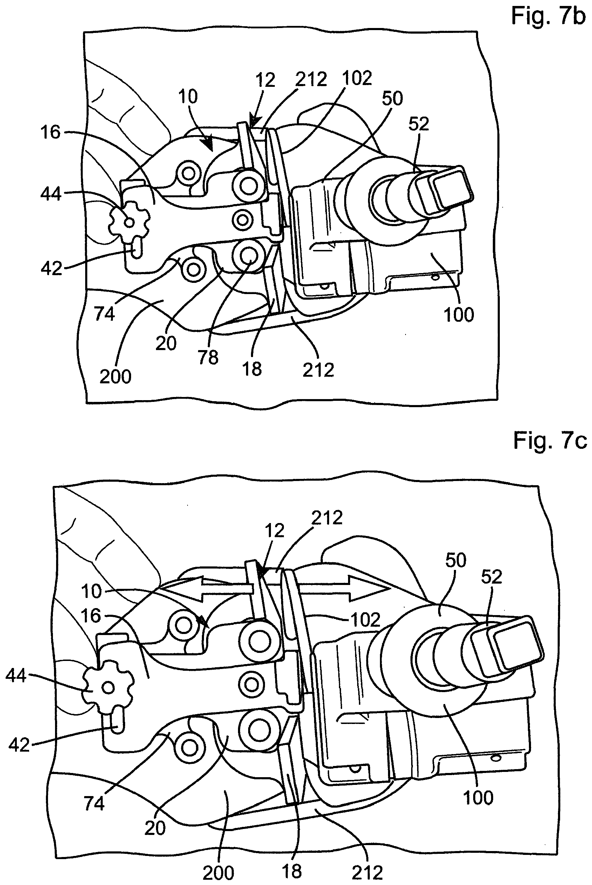

The intramedullary rod 14 is inserted and the knee joint 214 is placed in extension. As shown in Figure 7b, the support element 26 is connected to the intramedullary rod 14, and the femoral body 16 is engaged with the support element 26. The soft-tissue tensioner 52 is inserted between the tibia 216 and femur 204, so that the tibial platform 108 abuts the resected tibial surface 220, and the connector engager 104 engages the soft-tissue tensioning connector 20.

As shown in Figure 7c, the soft-tissue tensioner 52 is operated to provide tibio-femoral separation via the condylar locator 18 being forced away from the tibial platform 108. The medial and lateral collateral ligaments 212 and other ligamentous structures surrounding the knee are thus brought into tension.

Due to the pivoting of the condylar locator 18 relative to the soft-tissue tensioning element 50, the varus/valgus indicator 22 of the apparatus 12 indicates an angular alignment of the knee 200 based on the intramedullary rod 14 and/or condylar plane 222 and the distraction between the tibia 216 and the femur 204.

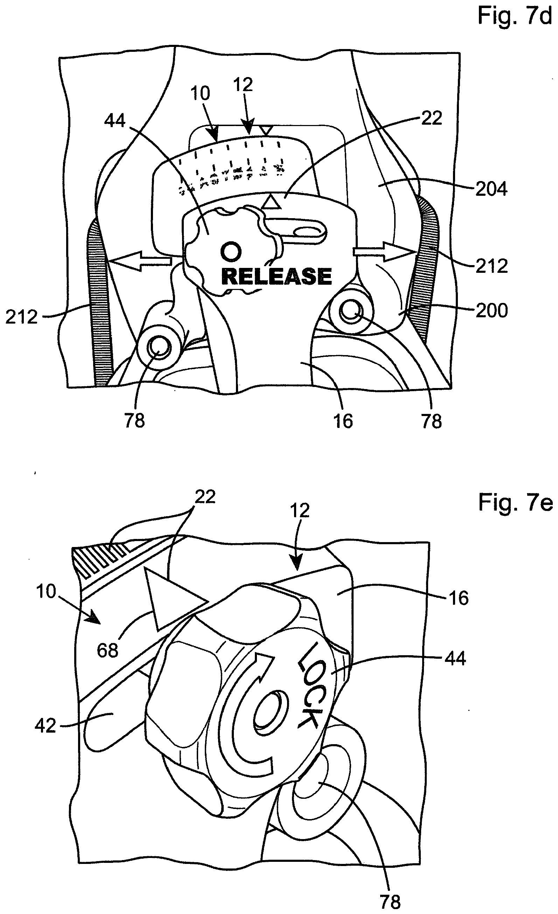

With the ligaments 212 in tension, soft-tissue release is performed until the required neutral alignment, typically being five degrees +/- say three degrees either side, is achieved as indicated by the varus/valgus indicator 22. See Figure 7d. This deviation of +/- three degrees would typically represent a knee with acceptable constitutional varus/valgus.

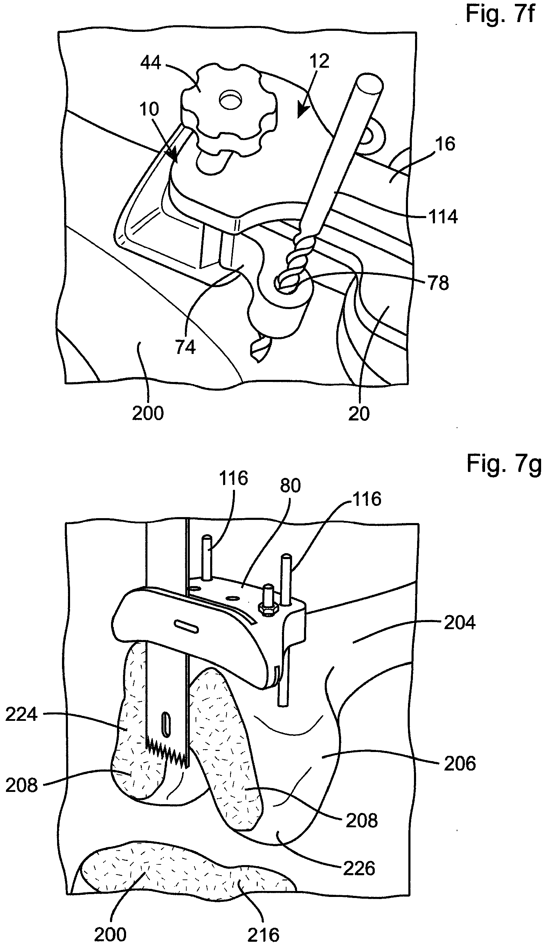

As shown in Figure 7e, the locking element 44 is activated to lock the femoral body 16 relative to the soft-tissue tensioning element 50. This locking holds the cutting-jig guide 74 in place, allowing a cutting tool 114, such as a surgical drill, to be received through the outboard cutting-jig locators 78, as shown in Figure 7f.

Due to the outboard cutting-jig locator 78 on one side being associated with the condylar platform 86 of the same side and movable in unison therewith whilst maintaining a fixed or substantially fixed separation, a cutting jig location can always be formed at a predetermined distance based on the most prominent distal condyle 208.

With the apparatus 12 then removed, as shown in Figure 7g, guide pins 116 are inserted at the cutting jig locations and the femoral cutting block or jig 80 is mounted on the distal end 206 of the femur 204. Distal femoral resection 224 can then take place as required.

The apparatus 12 of the present invention is also beneficial, since with the intramedullary rod 14 removed and the knee 200 in flexion, first part 10 of the apparatus 12 can be utilised to set an external rotation of the knee 200 by seating the condylar locator 18 on the medial posterior condyle 226 and using the cutting-jig locator 78 to provide a further cutting jig location in the distal end 206 of the femur 204. In this way, this sets the knee rotation and bone resections based on maintaining the medial collateral ligament isometry.

A further cutting jig can then be mounted on the resected face of the femur, and posterior and anterior resections of the femur can be undertaken, along with respective chamfers.

With the femoral resections completed, the tibial and femoral components of a knee prosthesis can be attached to the respective resected parts, and a meniscal component interposed therebetween in the normal manner.

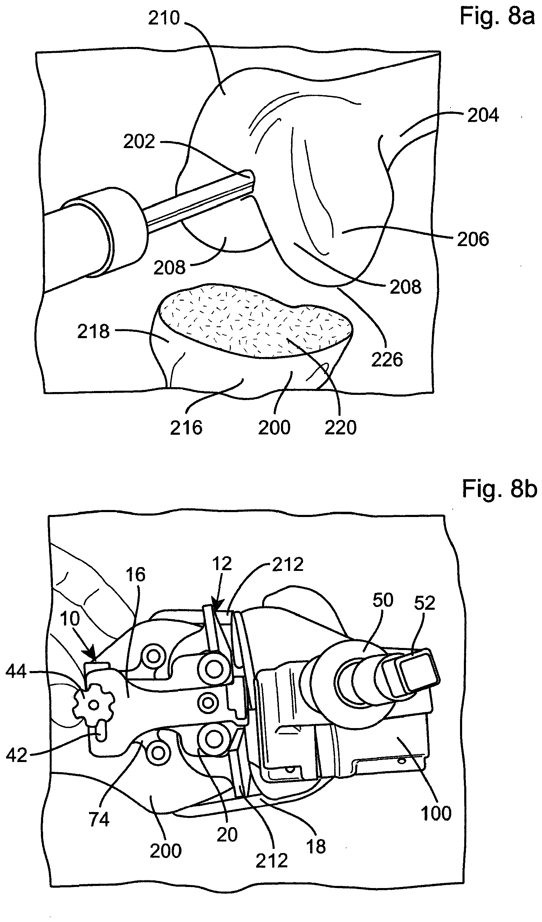

Turning now to Figures 8a to 8g, a constitutional varus/valgus technique is now described. Although described with reference to a varus knee, the same applies to a valgus knee.

For this technique, a pre-operative examination involving X-ray may be dispensed with.

With the knee 200 in flexion, the initial resection of the tibia 216 at right angles to the tibial mechanical axis in the coronal plane and with a three degree posterior slope in the sagittal plane is shown in Figure 8a, and is similar to that of the neutral leg alignment technique as described above. Although three degrees is suggested, this may be in the range of zero to ten degrees.

With the intramedullary canal 202 reamed and the intramedullary rod 14 inserted, the knee 200 is placed back into extension, the support element 26 is attached, and the femoral body 16 is connected thereto to extend anteriorly over at least a part of the distal end portion 210 of the femur 204. The soft-tissue tensioner 52 is inserted into the meniscal compartment of the joint and connected to the soft-tissue tensioning connector 20, as shown in Figure 8b.

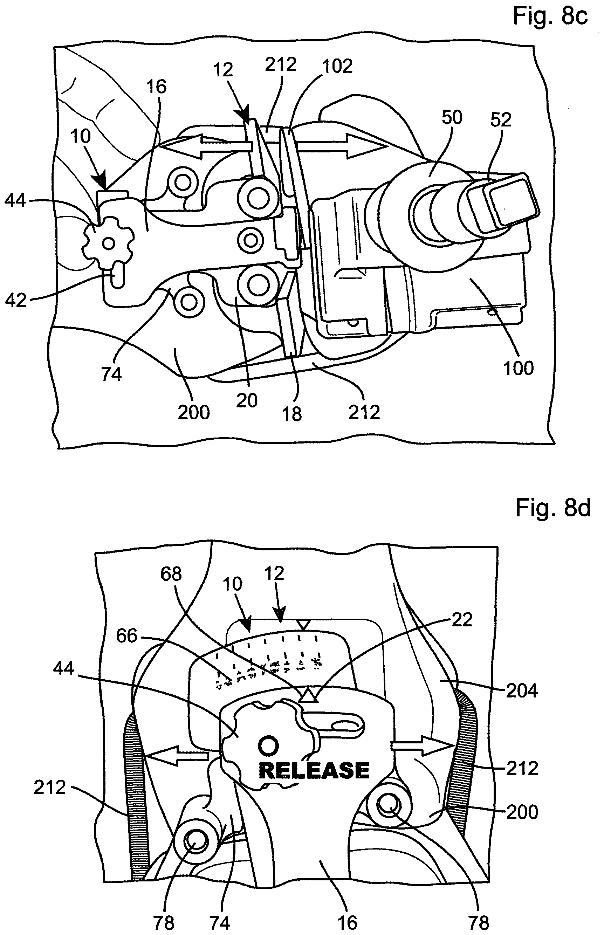

The soft-tissue tensioner 52 is operated to distract the tibia 216 and femur 204, by the tibial platform 108 seating on the resected tibial surface 220 and the soft-tissue tensioning connector 20 engaging the condylar locator 18. See Figure 8c.

With the knee 200 distracted, the varus/valgus indicator 22 is read. See Figure 8d. This reading is based on at least the medial and lateral collateral ligaments 212 being put under tension by the soft-tissue tensioner 52, thereby emulating the patient's normal upright stance.

With the true varus/valgus alignment being determined, a surgeon can then decide whether to perform ligament release to adjust this varus/valgus alignment to a desired value or to leave it as presented. For example, it is often preferable with modern techniques and understanding to maintain a patient's existing varus/valgus alignment to at least some extent, instead of attempting to fully correct the knee 200 to achieve a neutral leg alignment. This may be particularly beneficial for athletes or more active patients. It is now considered that the angle between the anatomical femoral axis and the tibial mechanical axis of between two degrees to eight degrees (being five degrees +/three degrees) is perfectly acceptable.

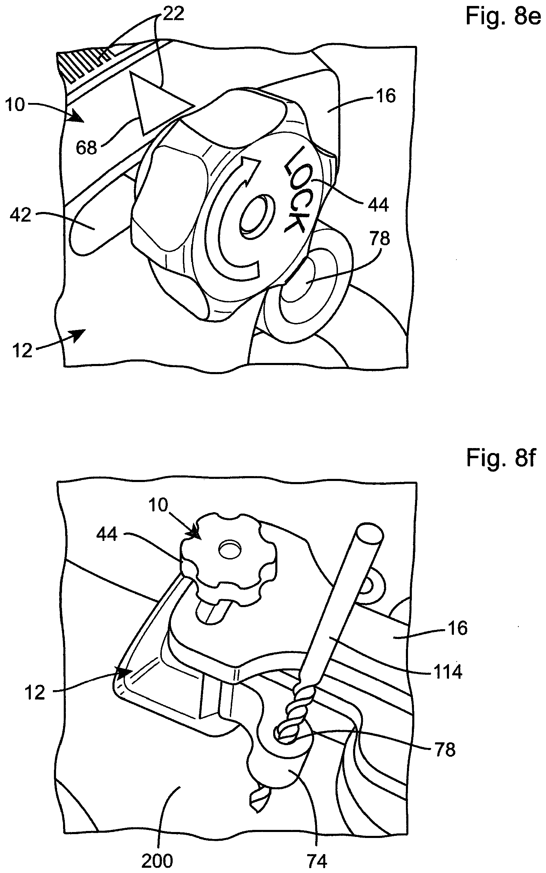

Once the varus/valgus alignment has been obtained, the locking element 44 is engaged, as shown in Figure 8e. This holds the soft-tissue tensioning connector 20 stationary relative to the femoral body 16.

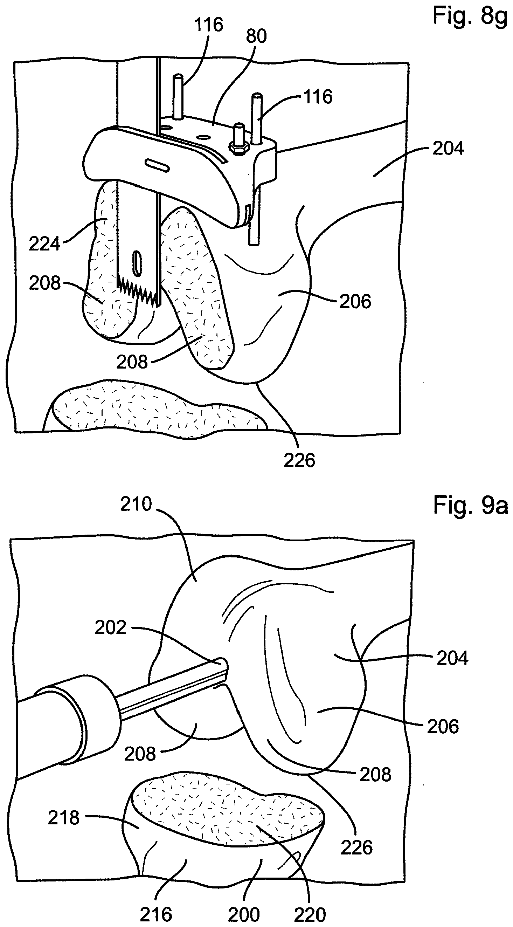

The steps of this technique shown by Figures 8f and 8g are the same as those described with reference to Figures 7f and 7g above. The engagement of the locking element 44 holds the cutting-jig guide 74 in place, allowing the cutting tool 114, such as a surgical drill, to be received through the outboard cutting-jig locators 78. See Figure 8f.

The cutting jig location is always formed at a predetermined distance from the distal condyles 208, based on the most prominent distal condyle and due to the synchronous movement of the outboard cutting-jig locator 78 and the respective condylar platform 86. With the apparatus 12 then removed, as shown in Figure 8g, guide pins 116 are inserted at the cutting jig locations and a femoral cutting block or jig 80 is mounted on the distal end 206 of the femur 204. Distal femoral resection 224 can then take place as required, along with setting external rotation of the knee 200 in the horizontal (transverse) plane, as necessary.

Referring now to Figures 9a to 9g, a modified varus/valgus technique is now described. Again, although described in relation to a varus knee 200, the same applies to a valgus knee 200.

This technique is similar to that of the constitutional varus/valgus technique described above with reference to Figures 8a to 8g, although a pre-operative examination using X-ray is required to determine an existing angle of the patient's knee 200 between the anatomical and mechanical axes of the femur 204.

As shown in Figure 9a and with either the left or right knee 200 in flexion, the proximal end 218 of the tibia 216 is resected perpendicularly or substantially perpendicularly to the mechanical axis of the tibia 216 in the coronal plane and with a three degrees slope in the sagittal plane. Although three degrees is suggested, this may be in the range of zero to ten degrees.

The intramedullary canal 202 is also reamed to accept the intramedullary rod 14.

The soft-tissue tensioning connector 20 is then locked via the locking element 44 relative to the femoral body 16, based on an angular alignment of the knee 200 decided during the pre-operative examination, described above. For example, if it is decided that the knee 200 should exhibit five degrees of femoral anatomical valgus, then the varus/valgus indicator 22 is set to five degrees and the locking element 44 is engaged.

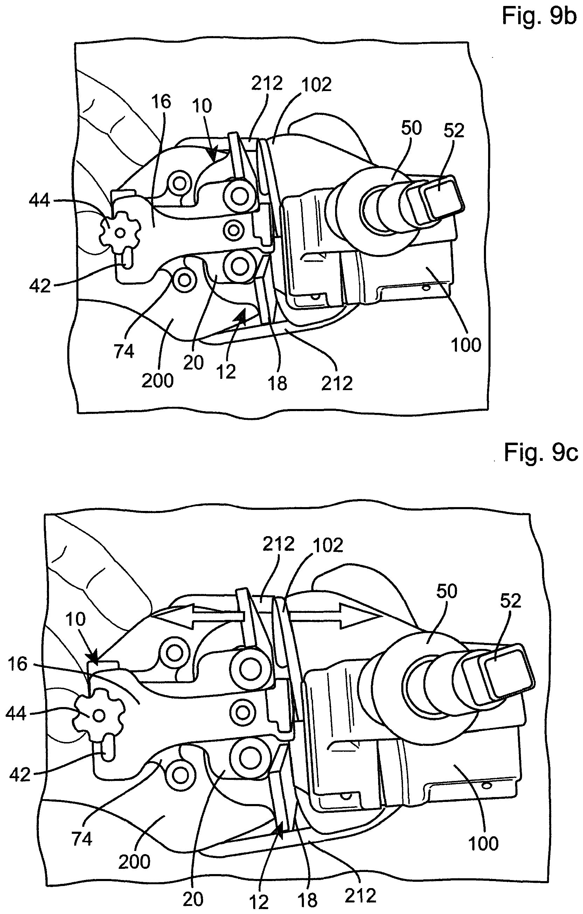

The apparatus 12 is then located on the knee 200 as described above with respect to the first and second techniques, see Figure 9b, and the knee 200 is distracted, as shown in Figure 9c.

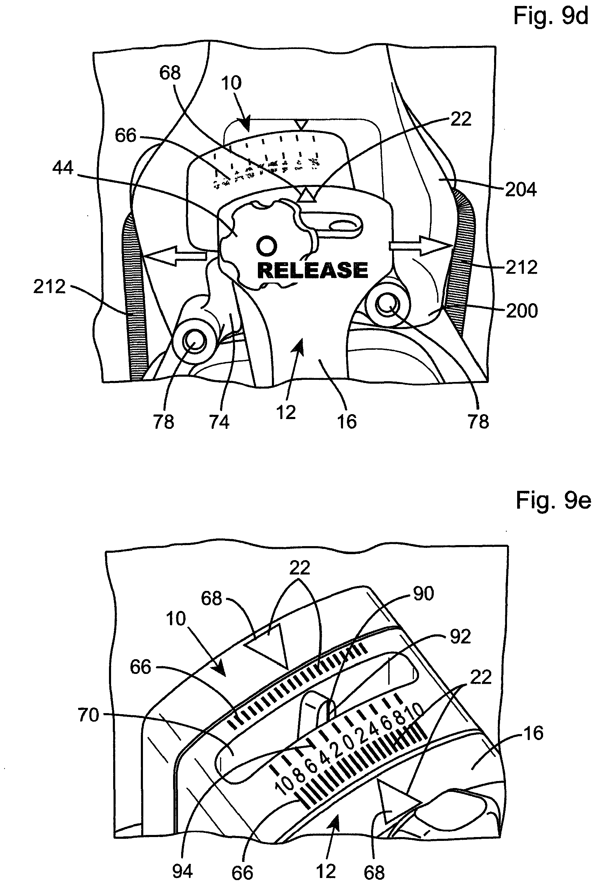

Due to the soft-tissue tensioning connector 20 being held stationary relative to the femoral body 16, as tibio-femoral separation is imparted by the apparatus 12, the condylar locator 18 tilts due to the tensioning of at least the medial and lateral collateral ligaments 212. See Figure 9d. The pointer arm 92 of the ligament tension indicator 90 indicates a balance of the ligaments on the ligament-tension scale 94 on the head element 56 of the soft-tissue tensioning connector 20. See Figure 9e. Soft-tissue release is then undertaken, if necessary, until the ligament tension indicator 90 typically reads zero to plus or minus three degrees.

Although not provided in this embodiment, it is feasible that the ligament tension indicator 90 may be lockable relative to the femoral body 16 and/or the soft-tissue tensioning connector 20. This may be provided by the aforementioned locking element 44, for example, by a locking pin being pushable further axially to engage not only the soft-tissue tensioning connector 20 but also the pointer arm 92 of the ligament tension indicator 90. Alternatively, a second separate locking element may be provided.

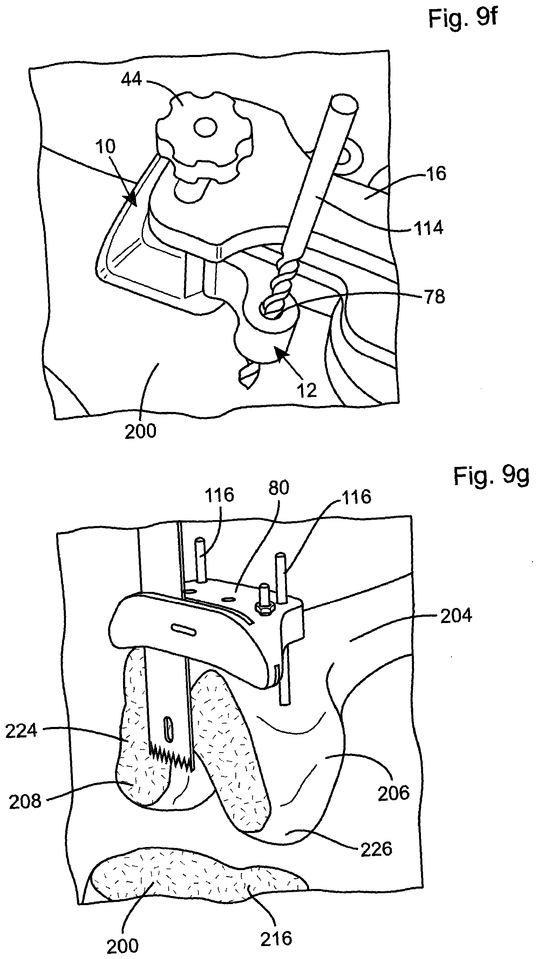

Figures 9f and 9g are similar to Figures 7f, 7g and 8f, 8g. The releasable locking holds the cutting-jig guide 74 in place, allowing the cutting tool 114, such as a surgical drill, to be received through the outboard cutting-jig locators 78. With the apparatus 12 then removed, guide pins 116 are inserted at the cutting jig locations and a femoral cutting block or jig 80 is mounted on the distal end 206 of the femur 204. Distal femoral resection 224 then takes place as required.

If required, again the first part 10 of the apparatus 12 can then be utilised to set the external rotation of the knee 200 by seating the condylar locator 18 on the medial posterior condyle 208 and using the cutting-jig locator 78 to provide a further cutting jig location.

With the femoral resections 224 completed, the tibial and femoral components of the knee prosthesis are attached to the respective resected parts, and the meniscal component is interposed therebetween in the normal manner.

A thickness of the prosthetic meniscal component is known, and for example may be 9 mm. By utilising a meniscal scale 118 on the soft-tissue tensioner 52, as shown in Figure 3, a thickness of the prosthetic meniscal component can be determined following distraction of the knee 200 and prior to distal femoral resection 224. The thickness is preferably from a base of the meniscal component to a lowermost point of its dished condylar seat. The precise positioning of the cutting jig 80 can thus be determined, and can be altered slightly from the cutting-jig guide 74 which is positioned by the movement of the condylar locator 18 and its location on the most prominent, or least worn, distal femoral condyle 208.

Although the soft-tissue tensioner is separate of the soft-tissue tensioning connector, these could be integrally formed together as one-piece, instead of being disengagable.

The ligament tension indicator and associated scale are optional, unless the modified constitutional varus/valgus technique is required.

The soft-tissue tensioning element may be or include shims for direct or indirect engagement with the condylar locator. This may thus dispense with the need for the soft-tissue tensioner, and possibly also the soft-tissue tensioning connector. The shims, which may be considered as spacers, may for example, include pockets for receiving the condylar platforms.

Although the condylar locator is preferably provided by two spaced apart condylar platforms, a single condylar abutting surface which spans between both medial and lateral condyles may be provided.

The apparatus of the present invention is particularly beneficial, as it provides a single apparatus which can be utilised with both left and right knees. Until now, separate devices had to be used for each knee. Furthermore, it is also possible to provide a single apparatus which can be used not only for angular alignment of the femur and tibia of both knees, when the knees are in extension, but also for the setting of external rotation, resection and ligament balancing of the knee utilising a datum taken from the medial posterior femoral condyle.

The embodiments described above are provided by way of examples only, and various other modifications will be apparent to persons skilled in the field without departing from the scope of the invention, as defined by the appended claims.

## Claims

1. Leg alignment apparatus (12) for use in total arthroplasty of left and right knees (200), the apparatus (12) comprising first and second parts (10, 100), the first part (10) comprising an intramedullary rod (14), a femoral body (16) for location on or adjacent to an anterior surface of a femur (204), a support element (26) which extends or is extendable from the femoral body (16) to the intramedullary rod (14) for holding the femoral body (16) stationary or substantially stationary relative to the intramedullary rod (14), a condylar locator (18) which is pivotable relative to the femoral body (16) and which is seatable on a distal end (206) of the femur (204), and a varus/valgus indicator (22), and the second part (100) comprising a soft-tissue tensioning element (50) to apply a separation between the femur (204) and the tibia (216), **characterised in that** the first part (10) includes a soft-tissue tensioning connector (20) which is pivotably mounted on the femoral body (16), the soft-tissue tensioning element (50) and the femoral body (16) being pivotably and releasably interconnected or interconnectable via the soft-tissue tensioning connector (20), and **in that** the varus/valgus indicator (22) indicates an angular alignment of the knee (200) based on the intramedullary rod (14) and the soft tissue tensioning connector (20),.

2. Leg alignment apparatus (12) as claimed in claim 1, wherein the in use soft-tissue tensioning element (50) applies a linear separation between the femur (204) and the tibia (216).

3. Leg alignment apparatus (12) as claimed in claim 1 or claim 2, wherein the condylar locator (18) is seatable on a distal end (206) of at least one of medial and lateral condyles (208).

4. Leg alignment apparatus (12) as claimed in claim 3, wherein the condylar locator (18) is seatable on a distal end (206) of both the medial and lateral condyles (208).

5. Leg alignment apparatus (12) as claimed in any one of claims 1 to 4, wherein the varus/valgus indicator (22) indicates an angular deviation of the knee (200) from neutral alignment.

6. Leg alignment apparatus (12) as claimed in any one of claims 1 to 5, wherein the condylar locator (18) includes medial and/or lateral elongate condylar platforms (86).

7. Leg alignment apparatus (12) as claimed in claim 6, wherein the condylar locator (18) is pivotable relative to the soft-tissue tensioning connector (20).

8. Leg alignment apparatus (12) as claimed in any one of claims 1 to 5, wherein the soft-tissue tensioning element (50) includes at least one shim element for distracting a tibia (216) and femur (204).

9. Leg alignment apparatus (12) as claimed in any one of claims 1 to 8, wherein the condylar locator (18) includes a ligament tension indicator (90) which is pivotable relative to the soft-tissue tensioning element (50).

10. Leg alignment apparatus (12) as claimed in any one of claims 1 to 9, wherein a longitudinal extent of the femoral body (16) is offset relative to a longitudinal extent of the intramedullary rod (14).

11. Leg alignment apparatus (12) as claimed in any one of claims 1 to 10, further comprising a cutting-jig guide (74) or a cutting jig (80) which is movable relative to the femoral body (16) and in unison with the condylar locator (18), so that a predetermined amount of bone is resectable from a distal end (206) of the femur (204) based on the most prominent femoral distal condyle (208).

12. Leg alignment apparatus as claimed in any one of claims 1 to 11, wherein the support element is integrally formed as one-piece with the intramedullary rod, and is angularly-offset relative to the longitudinal axis of the intramedullary rod for projection in a direction of an anterior surface of a femur.

## Patentansprüche

1. Beinausrichtungsvorrichtung (12) zur Verwendung in totaler Gelenkrekonstruktion von linken und rechten Knien (200), wobei die Vorrichtung (12) erste und zweite Teile (10, 100) umfasst, wobei das erste Teil (10) einen intramedullären Stab (14), einen Oberschenkelkörper (16) für die Lage auf oder angrenzend an eine vordere Fläche eines Oberschenkelknochens (204), ein Unterstützungselement (26), welches sich von dem Oberschenkelkörper (16) zu dem intramedullären Stab (14) zum Halten des Oberschenkelkörpers (16) stationär oder im Wesentlichen stationär relativ zu dem intramedullären Stab (14) erstreckt oder erstreckbar ist, einen kondylären Positionsanzeiger (18), welcher relativ zu dem Oberschenkelkörper (16) drehbar ist und welcher auf einem distalen Ende (206) von dem Oberschenkelknochen (204) aufsteckbar ist, und einen Varus/Valgus-Indikator (22) umfasst, und wobei der zweite Teil (100) ein Weichgewebe-Spannelement (50) umfasst, um eine Trennung zwischen dem Oberschenkelknochen (204) und dem Schienbein (216) anzulegen, **dadurch gekennzeichnet, dass** das erste Teil (10) einen Weichgewebe-Spannverbinder (20) einschließt, welcher drehbar auf dem Oberschenkelkörper (16) befestigt ist, wobei das Weichgewebe-Spannelement (50) und der Oberschenkelknochen (16) drehbar und lösbar miteinander verbunden sind oder miteinander verbindbar mittels dem Weichgewebe-Spannverbinder (20) sind, und dass der Varus/Valgus-Indikator (22) eine Winkelausrichtung von dem Knie (200) anzeigt, basierend auf dem intramedullären Stab (14) und dem Weichgewebe-Spannverbinder (20).

2. Beinausrichtungsvorrichtung (12) nach Anspruch 1, wobei das Weichgewebe-Spannelement (50) in Verwendung eine Trennung zwischen Oberschenkelknochen (204) und dem Schienbein (216) anlegt.

3. Beinausrichtungsvorrichtung (12) nach Anspruch 1 oder Anspruch 2, wobei der kondyläre Positionsanzeiger (18), auf einem distalen Ende (206) von zumindest einem von medialen und lateralen Kondylen (208) aufsteckbar ist.

4. Beinausrichtungsvorrichtung (12) nach Anspruch 3, wobei der kondyläre Positionsanzeiger (18), auf einem distalen Ende (206) von beiden, dem medialen und lateralen Kondylen (208), aufsteckbar ist.

5. Beinausrichtungsvorrichtung (12) nach einem der Ansprüche 1 bis 4, wobei der Varus/Valgus-Indikator (22) eine Winkelausrichtung von dem Knie (200) von der neutralen Ausrichtung anzeigt.

6. Beinausrichtungsvorrichtung (12) nach einem der Ansprüche 1 bis 5, wobei der kondyläre Positionsanzeiger (18), mediale und/oder laterale längliche Kondylenplattformen (86) einschließt.

7. Beinausrichtungsvorrichtung (12) nach Anspruch 6, wobei der kondyläre Positionsanzeiger (18) relativ zu dem Weichgewebe-Spannverbinder (20) drehbar ist.

8. Beinausrichtungsvorrichtung (12) nach einem der Ansprüche 1 bis 5, wobei das Weichgewebe-Spannelement (50) zumindest ein Ausgleichselement zum Ablenken eines Schienbeins (216) und Oberschenkelknochens (204) einschließt.

9. Beinausrichtungsvorrichtung (12) nach einem der Ansprüche 1 bis 8, wobei der kondyläre Positionsanzeiger (18) einen Bandspannungsindikator (90) einschließt, welcher relativ zu dem Weichgewebe-Spannelement (50) drehbar ist.

10. Beinausrichtungsvorrichtung (12) nach einem der Ansprüche 1 bis 9, wobei eine Längsstreckung von dem Oberschenkelkörper (16) relativ zu einer Längsstreckung von dem intramedullären Stab (14) versetzt ist.

11. Beinausrichtungsvorrichtung (12) nach einem der Ansprüche 1 bis 10, weiter umfassend eine Schneidvorrichtungsführung (74) oder ein Schneidwerkzeug (80), welches relativ zu dem Oberschenkelkörper (16) und einstimmig mit dem kondylären Positionsanzeiger (18) verschiebbar ist, sodass eine vorbestimmte Menge von Knochen von einem distalen Ende (206) von dem Oberschenkelknochen (204) resektierbar ist, basierend auf dem prominentesten distalen Oberschenkelgelenkkopf (208).

12. Beinausrichtungsvorrichtung (12) nach einem der Ansprüche 1 bis 11, wobei das Unterstützungselement als einteilig mit dem intramedullären Stab integral gebildet ist, und relativ zu der Längsachse von dem intramedullären Stab winklig versetzt ist zur Projektion in einer Richtung von einer vorderen Fläche eines Oberschenkelknochens.

## Revendications

1. Appareil d'alignement de jambe (12) pour utilisation en arthroplastie totale des genoux gauche et droit (200), l'appareil (12) comprenant une première et une seconde partie (10, 100), la première partie (10) comprenant une tige intramédullaire (14), un corps fémoral (16) pour localisation sur une surface antérieure d'un fémur (204) ou adjacente à celui-ci, un élément de support (26) qui s'étend ou peut s'étendre du corps fémoral (16) à la tige intramédullaire (14) pour maintenir le corps fémoral (16) immobile ou sensiblement immobile par rapport à la tige intramédullaire (14), un localisateur condylien (18) qui peut pivoter par rapport au corps fémoral (16) et qui peut se loger sur une extrémité distale (206) du fémur (204) et un indicateur de varus/valgus (22), et la seconde partie (100) comprenant un élément de tension de tissus mous (50) pour appliquer une séparation entre le fémur (204) et le tibia (216), **caractérisé en ce que** la première partie (10) comprend un raccord de tension de tissus mous (20) qui est monté à pivotement sur le corps fémoral (16), l'élément de tension de tissus mous (50) et le corps fémoral (16) étant ou pouvant être interconnectés à pivotement et de manière amovible via le raccord de tension de tissus mous (20), et **en ce que** l'indicateur de varus/valgus (22) indique un alignement angulaire du genou (200) sur la base de la tige intramédullaire (14) et du raccord de tension de tissus mous (20).

2. Appareil d'alignement de jambe (12) selon la revendication 1, dans lequel l'élément de tension de tissus mous (50) en usage applique une séparation linéaire entre le fémur (204) et le tibia (216).

3. Appareil d'alignement de jambe (12) selon la revendication 1 ou la revendication 2, dans lequel le localisateur condylien (18) peut être logé sur une extrémité distale (206) d'au moins un des condyles médian et latéraux (208).

4. Appareil d'alignement de jambe (12) selon la revendication 3, dans lequel le localisateur condylien (18) peut être logé sur une extrémité distale (206) des condyles à la fois médian et latéraux (208).

5. Appareil d'alignement de jambe (12) selon l'une quelconque des revendications 1 à 4, dans lequel l'indicateur de varus/valgus (22) indique une déviation angulaire du genou (200) vis-à-vis d'un alignement neutre.

6. Appareil d'alignement de jambe (12) selon l'une quelconque des revendications 1 à 5, dans lequel le localisateur condylien (18) comprend des plateformes condyliennes médiane et/ou latérales allongées (86).

7. Appareil d'alignement de jambe (12) selon la revendication 6, dans lequel le localisateur condylien (18) peut pivoter par rapport au raccord de tension de tissus mous (20).

8. Appareil d'alignement de jambe (12) selon l'une quelconque des revendications 1 à 5, dans lequel l'élément de tension de tissus mous (50) comprend au moins un élément de cale pour distraire un tibia (216) et un fémur (204).

9. Appareil d'alignement de jambe (12) selon l'une quelconque des revendications 1 à 8, dans lequel le localisateur condylien (18) comprend un indicateur de tension de ligaments (90) qui peut pivoter par rapport à l'élément de tension de tissus mous (50).

10. Appareil d'alignement de jambe (12) selon l'une quelconque des revendications 1 à 9, dans lequel une extension longitudinale du corps fémoral (16) est décalée par rapport à une extension longitudinale de la tige intramédullaire (14).

11. Appareil d'alignement de jambe (12) selon l'une quelconque des revendications 1 à 10, comprenant en outre un guide gabarit de coupe (74) ou un gabarit de coupe (80) qui est mobile par rapport au corps fémoral (16) et à l'unisson avec le localisateur condylien (18) de sorte qu'une quantité prédéterminée d'os puisse être réséquée d'une extrémité distale (206) du fémur (204) sur la base du condyle distal fémoral le plus proéminent (208).

12. Appareil d'alignement de jambe (12) selon l'une quelconque des revendications 1 à 11, dans lequel l'élément de support est formé d'un seul tenant avec la tige intramédullaire et est décalé au plan angulaire par rapport à l'axe longitudinal de la tige intramédullaire pour faire saillie dans la direction d'une surface antérieure d'un fémur.
